(19) **Europäisches Patentamt / European Patent Office / Office européen des brevets**

(11) **EP 4 471 797 A1**

(12) **EUROPEAN PATENT APPLICATION**
published in accordance with Art. 153(4) EPC

(43) Date of publication:
04.12.2024 Bulletin 2024/49

(21) Application number: 23747402.8

(22) Date of filing: 27.01.2023

(51) International Patent Classification (IPC):
*G16H 50/20* (2018.01)  *G16B 40/20* (2019.01)
*G16B 15/30* (2019.01)  *A61B 5/00* (2006.01)
*A61B 5/318* (2021.01)  *G16H 10/20* (2018.01)
*G16H 20/10* (2018.01)  *G16H 50/50* (2018.01)
*G01N 33/68* (2006.01)

(52) Cooperative Patent Classification (CPC):
A61B 5/00; A61B 5/318; G01N 33/68; G06N 3/04;
G06N 3/08; G06N 20/00; G16B 15/30; G16B 40/20;
G16H 10/20; G16H 20/10; G16H 50/20;
G16H 50/50

(86) International application number:
PCT/KR2023/001315

(87) International publication number:
WO 2023/146361 (03.08.2023 Gazette 2023/31)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 28.01.2022 KR 20220013491

(71) Applicant: **Seoul National University Hospital**
**Seoul 03080 (KR)**

(72) Inventors:
• **KIM, Joonghee**
  **Seongnam-si, Gyeonggi-do 13620 (KR)**
• **CHO, Youngjin**
  **Seongnam-si, Gyeonggi-do 13620 (KR)**

(74) Representative: **V.O.**
**P.O. Box 87930**
**2508 DH Den Haag (NL)**

(54) **ARTIFICIAL INTELLIGENCE-BASED BIOMARKER SELECTION DEVICE AND METHOD**

(57)    The embodiments relate to an artificial intelligence-based biomarker selection device and method, the artificial intelligence-based biomarker selection device comprising: an acquisition unit for acquiring biodata; an encoder for receiving the biodata and calculating a numeric vector including one or more elements which are biomarker candidates; and a biomarker screening unit for screening biomarkers from the one or more elements of the numeric vector. The device and method of the embodiments may be used for discovering various biomarkers usable for drug discovery, such as the development of, for example, a new cardiovascular drug, or for prognosis prediction. Accordingly, the device and method may be effectively used for a pharmaceutical platform for new drug development, or for a research platform for precision medicine, disease diagnosis, treatment optimization, etc.

FIG. 1

Biodata

| Biodata | — 10 |
| Encoder | — 20 |
| Biomarker screening unit | — 30 |

prognostic biomarker
predictive biomarker
monitoring biomarker
surrogate biomarker

⋮

## Description

### Technology Field

This specification relates to an artificial intelligence-based biomarker selection device and method, and to an artificial intelligence-based biomarker selection device and method, which can develop companion diagnostic biomarkers based on patient-derived information for new drug development, precision medicine, disease diagnosis, and treatment optimization.

### Cross-reference to related application

This application claims priority to Republic of Korea Provisional Patent Application No. 10-2022-0013491 filed on January 28 , 2022 , the entire contents of which are incorporated by reference into this application.

### Background Art

Companion diagnostic is a test used to determine whether a patient with a specific disease can be treated with a specific drug through biomarker evaluation .

Here, the biomarker includes a predictive biomarker that can distinguish between responders and non-responders to a specific drug or prognostic biomarkers that can predict the prognosis of a disease and indicates the extent of disease progression or whether treatment is needed, etc.

Existing traditional biomarkers are usually selected using technologies such as polymerase chain reaction (PCR), insituhybridization (ISH), next generation sequencing (NGS), and immunohistochemistry (IHC).

However, most companion diagnostics are used only in relation to serious diseases such as cancer and the drugs that treat them, and there are often no biomarkers for other diseases or drugs.

For example, in the case of cardiovascular drugs, they are widely prescribed drugs and most patients take them throughout their lives, so there is a need for companion diagnosis, but there is no effective biomarker.

One of the reasons why effective biomarkers are absent or attempts to find such biomarkers are not made for certain diseases is that it is very difficult and time-consuming to select biomarkers using traditional methods as mentioned above.

### Detailed Description of the Invention

### Technical Solution

In one aspect, embodiments of the present application relate to an artificial intelligence-based biomarker selection device and method that can develop companion diagnostic biomarkers that can be utilized in new drug development, precision medicine, disease diagnosis, and treatment optimization for various diseases, including diseases for which existing biomarkers are absent, such as cardiovascular diseases.

### Summary of the Invention

In embodiments of the present application, as an artificial intelligence-based biomarker selection method, it provides an artificial intelligence-based biomarker selection method and a computer-readable recording medium recording a program for performing the same or a computer program stored in a computer-readable recording medium, which comprises an encoding step of encoding biodata to calculate a numeric vector including one or more elements that are biomarker candidates; and a biomarker screening step of screening biomarkers from one or more elements of the numeric vector.

Additionally, in embodiments of the present application, as an artificial intelligence-based biomarker selection device, to provides an artificial intelligence-based biomarker selection device includes an acquisition unit that acquires biodata; an encoder that receives the biodata and calculating a numeric vector including one or more elements which are biomarker candidates; and a biomarker screening unit for screening biomarkers from one or more elements of the numeric vector.

### Advantageous Effects

In one aspect, according to the embodiments of the present application, it can be used to discover various biomarkers that can be used for drug discovery or prognosis prediction, for example, in the development of new drugs for the cardiovascular system. Accordingly, it can be effectively used as a pharmaceutical platform for new drug development or a research platform for precision medicine, disease diagnosis, and treatment optimization, etc.

The effects of this application are not limited to the effects mentioned above, and other effects that are not mentioned can be clearly understood by a person skilled in the art from the description of the claims.

## Brief Description of the Drawings

In order to more clearly describe the exemplary embodiments of the present application, drawings necessary in the description of the embodiments are briefly introduced below. It should be understood that the drawings below are for illustrative purposes of the embodiments of the present specification only and not for limiting purposes. Additionally, some elements that have been subject to various modifications such as exaggeration and omission in the drawings below may be illustrated for clarity of explanation.

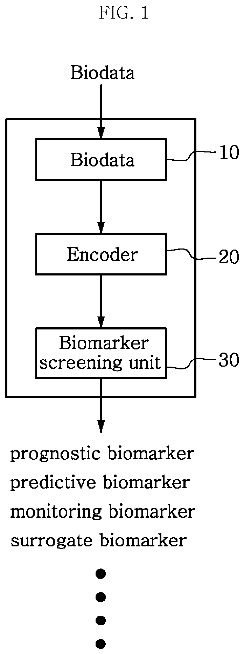

FIG. 1 is a schematic diagram showing the configuration of an an artificial intelligence-based biomarker selection device according to one aspect of the present application.

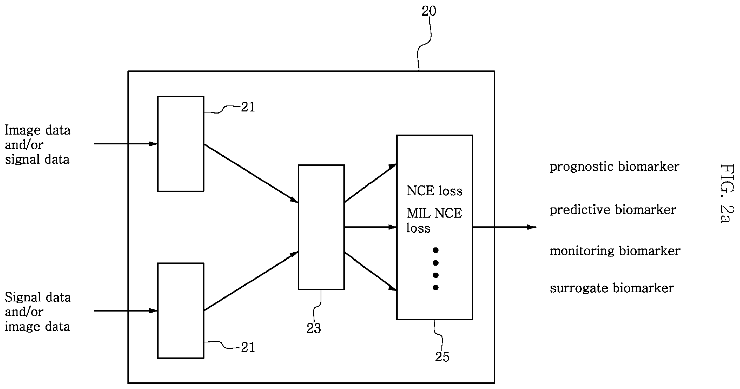

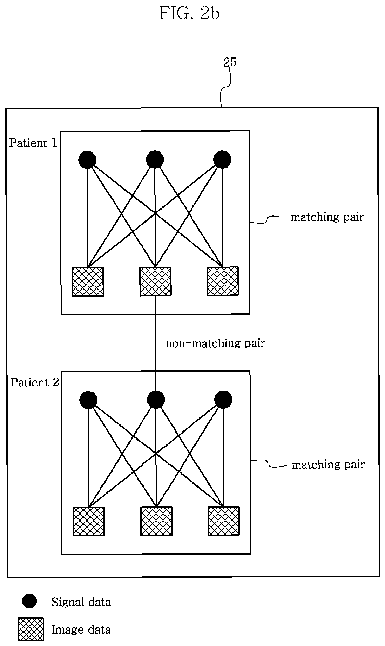

FIG. 2a is a schematic diagram showing the encoder architecture of an artificial intelligence-based biomarker selection device in one embodiment of the present application, and FIG. 2b is a schematic diagram illustrating the process of finding matching pairs and non-matching pairs in the common space of FIG. 2a.

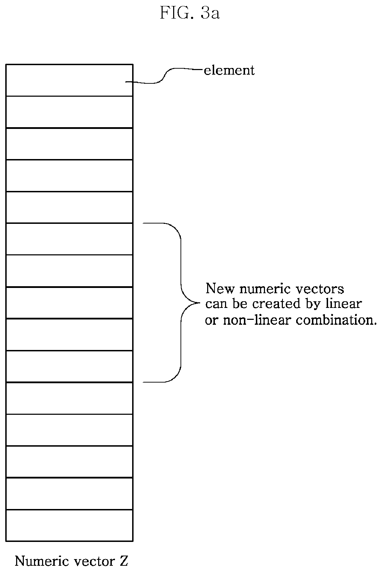

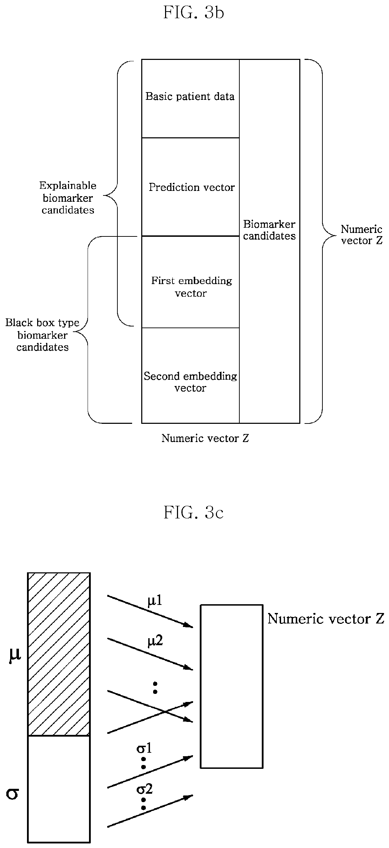

FIG. 3a is a schematic diagram showing a numeric vector including one or more elements that are biomarker candidates in one embodiment of the present application, and FIG. 3b is an explanatory diagram explaining the configuration and role of the corresponding numeric vector. FIG. 3c is a schematic diagram illustrating a process for improving the embedding quality of each element of a numeric vector in one embodiment of the present application.

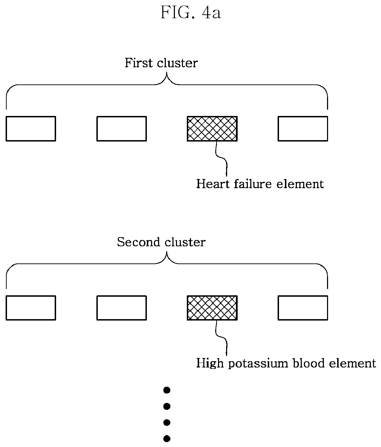

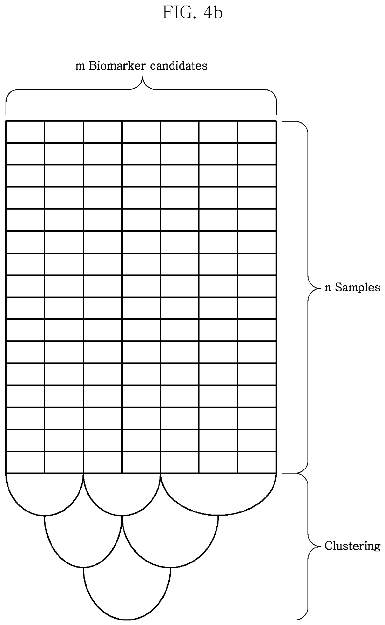

FIG. 4 is a schematic diagram illustrating selection by clustering during a biomarker screening process in one embodiment of the present application. FIG. 4a is a diagram illustrating each individual cluster, and FIG. 4b is a diagram illustrating a numeric array (matrix) composed by extracting m candidate biomarkers from n subjects (or samples).

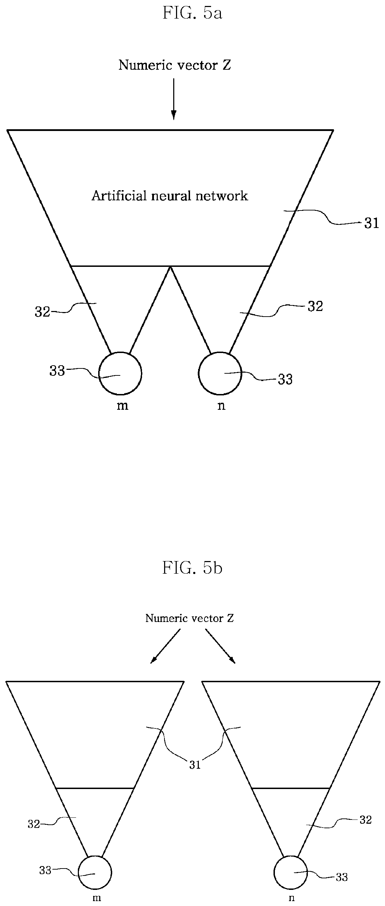

FIG. 5 is a schematic diagram of a process for generating an effect modifier biomarker m that indicates the effect of a drug or treatment during a biomarker screening process using an artificial neural network in one embodiment of the present application, and a biomarker n that is unrelated to the effect modification. FIG. 5a illustrates a case where a numeric vector Z passes through one neural network, and FIG. 5b illustrates a case where a numeric vector Z passes through different neural networks.

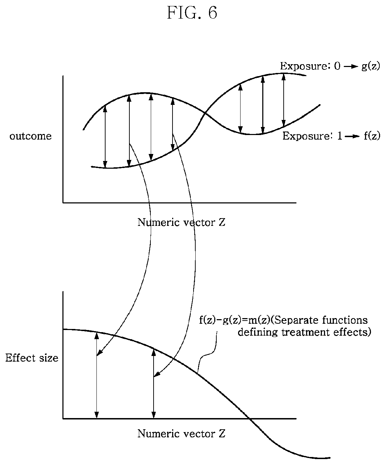

FIG. 6 is a schematic diagram illustrating a process for measuring effect modification during a biomarker screening process in one embodiment of the present application.

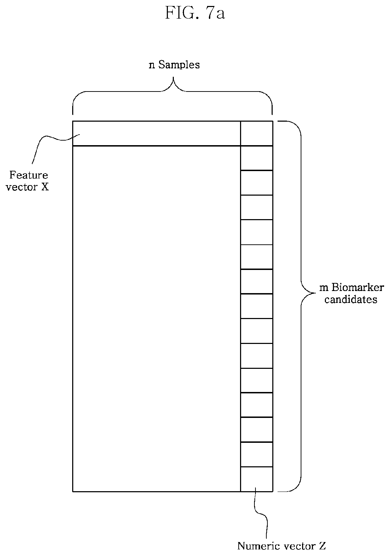

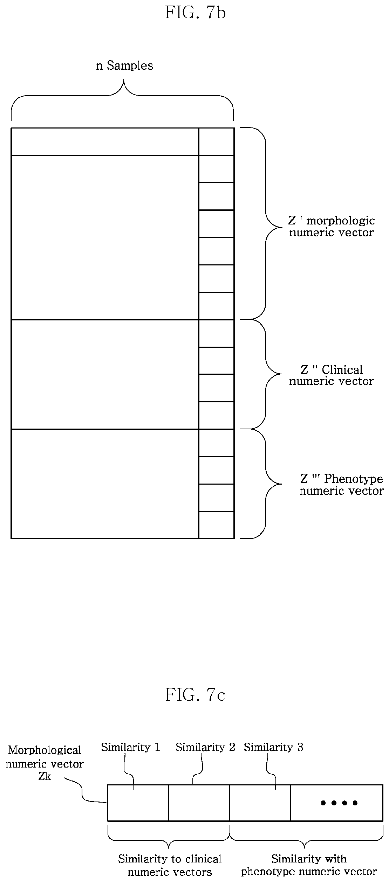

FIG. 7 is a schematic diagram showing a process for finding attributes of a biomarker during a biomarker screening process in one embodiment of the present application. FIG. 7a is a schematic diagram summarizing the characteristics of a numeric vector Z, in which m biomarker candidates belonging to the numeric vector Z in an m x n matrix each have n attribute vectors X. FIG. 7b is a diagram explaining a case where all biomarker candidates are divided into elements of a morphological numeric vector Z', a clinical numeric vector Z", and a phenotype numeric vector Z‴, and FIG. 7c is a diagram illustrating obtaining an attribute vector of a morphological numeric vector based on similarity.

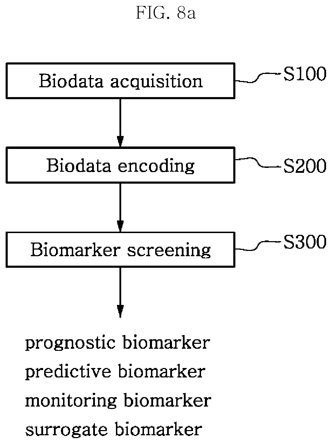

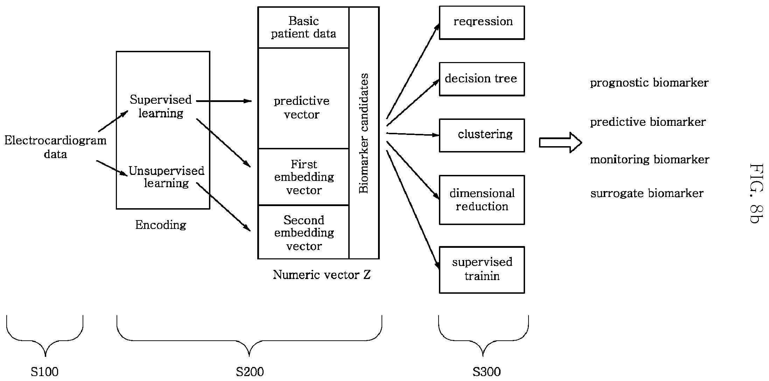

FIG. 8 is a schematic diagram illustrating an artificial intelligence-based biomarker selection method in one embodiment of the present application. FIG. 8a is a schematic diagram illustrating each step of the method, and FIG. 8b is a schematic diagram showing a numeric vector, which are biomarker candidates obtained in the process of FIG. 8a, and selecting a biomarker therefrom to obtain a biomarker, particularly in the case of using electrocardiogram data.

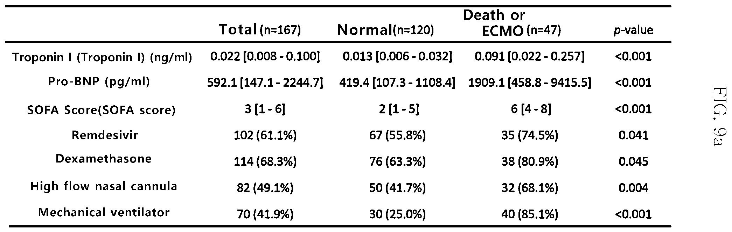

FIG. 9a to FIG. 9c are tables and graphs showing experimental data that developed a new electrocardiogram biomarker for predicting the mortality risk of COVID-19 infection patients using a numeric vector Z extracted from an existing electrocardiogram analysis encoder according to an experimental example of the present invention and evaluated its sensitivity, specificity, etc.

## Detailed Description of the Embodiments

Hereinafter, some embodiments of the present application will be described in detail with reference to the exemplary drawings. In adding reference numerals to components in each drawing, identical components may have the same reference numerals as much as possible even if they are shown in different drawings. In addition, when describing the present embodiments, if it is determined that a detailed description of a related known configuration or function may obscure the gist of the present technical idea, the detailed description may be omitted.

## Definition of terms

When "includes," "has," "consists of," etc. mentioned in this specification are used, other parts may be added unless "only" is used. When a component is expressed in the singular, it can also include the plural, unless specifically stated otherwise.

Additionally, in describing the components of this application, terms such as first , second , A, B, (a), (b), etc. may be used. Unless otherwise specified, these terms are only used to distinguish the component from other components, and the nature, sequence, order, or number of the components are not limited by the term.

In this specification, 'learning' or 'study' is a term referring to performing machine learning through procedural computing.

In this specification, network refers to a neural network of a machine learning algorithm or model.

In this specification, the terms "unit," "module," "device," or "system" are intended to refer to a combination of hardware as well as software driven by the hardware. For example, the hardware may be a data processing device including a Central Processing Unit (CPU), a Graphic Processing Unit (GPU), or other processor.

In this specification, a biomarker refers to a digital biomarker.

In this specification, a biomarker candidate refers to a numeric vector or elements of a numeric vector before being selected as a biomarker.

In this specification, biodata refers to patient-derived information that can be used for new drug development, precision medicine, disease diagnosis and treatment optimization, etc. For example, it may be a bio signal such as an electrocardiogram, but is not limited to this.

In this specification, modality may refer to formal characteristics such as, for example , a waveform signal or image of a bio signal.

In this specification, a platform or platform system may mean at least a part of an artificial intelligence-based biomarker selection device of the present invention. For example, a pharmaceutical platform for new drug development, etc. or a research platform for various types of precision medicine, disease diagnosis or treatment optimization, etc. may include at least a portion of the artificial intelligence-based biomarker selection device of the present invention or may be the device itself.

In this specification, common space refers to a shared coordinate system that can express and compare the similarity between numeric vectors, and refers to a dimensional space into which numeric vectors are projected to find pairs between numeric vectors.

Various data mining techniques, such as clustering and dimensionality reduction, mentioned in this specification encompass all data mining methodologies applicable based on vectors made of real numbers and are not limited to the specific examples presented.

## Description of Exemplary Implementations

FIG. 1 is a schematic diagram showing the configuration of an artificial intelligence-based biomarker selection device according to one aspect of the present application.

Referring to FIG. 1, an artificial intelligence-based biomarker selection device may include an acquisition unit 10 that acquires biodata, an encoder 20 for receiving biodata from the acquisition unit 10 and calculating a numeric vector including one or more elements that are biomarker candidates, and a biomarker screening unit 30 for screening biomarkers from one or more elements of the numeric vector.

The acquisition unit 10 acquires biodata, which is one or more patient-derived information, from an acquisition device (not shown).

The biodata may be two or more biodata of different or identical modalities. In one example, the biodata may include at least one of, for example, waveform signal data and image data. When the biodata is an electrocardiogram, the biodata as input data may include at least one of an electrocardiogram signal that is a one-dimensional single-channel or multi-channel signal and an electrocardiogram image in which the corresponding electrocardiogram signal is depicted on a two-dimensional plane.

In one example, the signal is in the form of a two-dimensional array, for example, CxT (the number of each input lead (channel) x the number of measurement values for each channel), and the image may be an image including all lead channels, or a patch image for each lead channel by cropping for each lead channel, and may be a black-and-white image of one or more lead channels, or a three-dimensional array in the form of CxWxH (number of channels x number of horizontal pixels x number of vertical pixels) having three channels: Red (R), Green (G), and Blue (B).

Meanwhile, the biodata may be any type of patient-derived information. As will be described later in an exemplary embodiment, it may be representatively electrocardiography (ECG) data, but is not limited thereto, and may include various time-series bio-signals such as electroencephalogram (EEG), electromyogram (EMG), electrocoulography (EOG), etc. In addition, it can have waves, images, sounds, and any other form of modality, and is not limited to a specific modality. In addition, biodata may also include various basic patient data, such as the patient's gender, age, and existing disease history.

The encoder 20 can be trained through supervised learning or self-supervised learning to provide a numeric vector with one or more elements that are biomarker candidates, and can include various neural network structures suitable for providing the numeric vector (hereinafter, referred to as numeric vector Z).

In one example, the encoder 20 may include a clinical encoder and at least one of a clinical meaning and morphologic encoder.

The clinical encoder may be configured to receive biodata as input and output a first numeric vector including one or more elements associated with clinical meaning through supervised learning related to a task having clinical meaning.

The morphological encoder may receive biodata and calculate a second numeric vector including one or more elements associated with morphological characteristics of the biodata. The corresponding morphological encoder may be trained by unsupervised learning.

The first numeric vector Z1 that has passed through the clinical encoder described above and the second numeric vector Z2 that has passed through the morphological encoder can be transformed for the same biodata input through a stochastic process. For example, this process can include test-time random augmentation or Monte-Carlo dropout (or drop connect or a similar known technique), Bayesian layer, etc.

In one example, the encoder 20 may also provide a new numeric vector by concatenating the first numeric vector Z1 and the second numeric vector Z2. To this end, the neural network of the encoder 20 may be trained by multi-task learning that integrates supervised learning and unsupervised learning.

Additionally, in one example, at least one of the mean, range and standard deviation (SD) of some or all of one or more elements of each numeric vector 21, Z2 repeatedly generated from the encoder 20 may be included in the biomarker candidates.

Furthermore, the encoder 20 can be trained and used by additionally projecting the generated numeric vectors onto a common space and finding pairs based on the similarity between the numeric vectors.

For example, the encoder 20 can be trained in a way to find a positive fair based on the similarity between the numeric vectors by projecting the calculated numeric vector on a common space using an additional artificial neural network trained together.

Here, the positive pair can be said to be a matching pair within a defined time range (window frame) of the same subject.

And/or, the encoder 20 can be trained in a way to find a negative fair based on the similarity between the numeric vectors by projecting the calculated numeric vector on a common space.

Here, a negative pair can be a pair outside the given time window frame of the same subject or a pair between different subjects. This can also be referred to as a non-matching pair.

Here, the fixed time range (window frame) can be set to, for example, a certain number of minutes, a certain number of hours, a certain number of days, etc., and the time range size can be adjusted according to the condition of the subject according to the main projection or anchor (i.e., the numeric vector that is the main one among the pairs) projected on the common space. That is, the fixed time range (window frame) can have an adaptive time window size. For example, when electrocardiogram data obtained from an emergency room patient is input and the encoder searches for a pair, the time window size can be narrowed, and when electrocardiogram data obtained from an outpatient is used, the time window size can be widened.

Additionally, the encoder 20 can further use the calculated numeric vector for supervised learning task training to have clinical meaning.

FIG. 2a is a schematic diagram showing the encoder architecture of an artificial intelligence-based biomarker selection device in one embodiment of the present application, and FIG. 2b is a schematic diagram illustrating the process of finding matching pairs [positive pair] and non-matching pairs [negative pair] in the common space of FIG. 2a.

As shown in FIGS. 2a and 2b , the encoder 20 may be structured with one or more inspection methods, i.e., a bottom layer 21 for each modality and a common head 23, and may be trained in a manner of projecting a numeric vector scattered from the common head 23 onto a common space 25 to find a matching pair and/or a non-matching pair.

The bottom layer 21 may be a layer for each modality, and receives each biodata and outputs a series of vectors to be entered into the common head 23.

In one example, when providing a plurality of signals and/or images to the bottom layer, a patching process and position embedding can be applied to the original input as part of the preprocessing process. The position embedding can be position embedding according to T (time) for signals, and according to H (height) and W (width) for images.

Here, the bottom layer 21 corresponds to various artificial neural networks having suitable structures for each modality. These include multi-layer perceptron (MLP, hereinafter referred to as MLP), convolutional neural network (CNN, hereinafter referred to as CNN), transformer, recurrent neural network (RNN, hereinafter referred to as RNN), and various modifications thereof, and are not limited to a specific structure of the artificial neural network. The common head 23 layer may have, for example, a transformer neural network structure and/or an MLP structure.

The vectors passing through the common head 23 layer can be projected onto a common space, and trained in a way that positive pairs and/or negative pairs are found based on the similarity between numeric vectors.

In one example, the encoder 20 can be further trained to have clinical meaning by performing a supervised learning task in addition to training to find pairs. That is, in addition to matching biodata such as electrocardiograms with data of the same person within a given time range, it can also be trained to predict the patient's condition and diagnosis at the time of examination, so that the encoded results can perform specific tasks together to have clinical meaning.

In one example, the supervised learning task is a task related to prediction, and may be a task of predicting one or more of binary, multi-category, and numeric values simultaneously or sequentially. For example, it may be a task of predicting the presence or absence of a patient's disease (binary), a classification task (multi-category), or a task of predicting a specific measurement value (numerical).

In one example, when trained to find a pair in the common space 25 , loss function may include Noise-Contrastive-Estimation (NCE) loss .

Additionally, in another example, the loss function may additionally use MIL NCE (Multiple-Instance-Learning-NCE) loss in addition to NCE loss, and/or may additionally include supervised task loss if performing a supervised task. The total loss in this case can be expressed as in the following [Equation 1].

Total loss = NCE Loss + $\lambda$MILNICE Loss + $\tau$ Supervised Learning Task Loss [Equation 1]

In the [Equation 1], $\lambda$ and $\tau$ can be adjusted and used as a hyperparameter, and each can be 0.

Meanwhile, the positive pair or negative pair may include one or more of a pair between signals; a pair between images; and a pair between signals and images. Here, a signal refers to single-channel or multi-channel one-dimensional time series data, and an image refers to a waveform image that expresses this in various formats on a two-dimensional plane.

In one example, the signal and/or image may be data augmented. Data augmentation is the application of various transformations to the original input data, and any known data augmentation technique such as rotation, flip, channel drop, cut out, cut mix, etc. may be used.

When training positive ECG pairs of mutually identical formats (e.g., signal-signal, image-image) to be located close to each other in a common space, training is possible in a way that makes the first and second encoders identical or similar. Specifically, a specific encoder (first encoder) in a format capable of processing an input signal, a second encoder having the same structure and parameters (weight-sharing) as the first encoder, or a second encoder (momentum encoder) created by applying a weighted average to the most recent parameter values of the first encoder during the training process can be used.

In one example, the encoder 20 includes a first encoder that receives a first bio-data set that has been subjected to data augmentation processing, and a second encoder that receives a second bio-data set that has been subjected to data augmentation processing. The first bio-data set and the second bio-data set may be random augmentation-processed signals or images of the same electrocardiogram or a set time range of the same patient (subject).

Here, the first encoder may be any neural network that converts an input value into a numeric vector, and the second encoder may be a momentum encoder that shares the same weight as the first encoder or averages the temporal changes of the first encoder.

Here, the first encoder may or may not further include an MLP layer. The MLP layer may be used to transform the produced numeric vector into a new numeric vector.

Additionally, the second encoder may or may not perform clustering, which converts the produced numeric vector into a limited number of embedded numeric vectors. The clustering is a task of selecting the embedded numeric vector closest to the produced numeric vector, and serves to replace the original numeric vector output with the closest embedded numeric vector (representative value).

For the numeric vectors that have gone through this process, a gradient flow process can be performed to adjust the parameters of the encoder neural network using a similarity function and/or a dissimilarity function as a loss function. For example, when a dissimilarity function is used, the gradient flow can be sent to both the first encoder and the second encoder or to the first encoder to adjust the parameters of each encoder.

Here, the similarity function can be used between positive pairs, and the dissimilarity function can be used between negative pairs.

Additionally, the above-described supervised task can be additionally performed to impart clinical meaning to the gradient flow process described above.

The total loss when using similarity function and/or dissimilarity function and additionally performing supervised task can be expressed as follows [Equation 2]

Total Loss = $\gamma$ similar loss + $\lambda$ dissimilarity loss + $\tau$ supervised task loss [Equation 2]

In the [Equation 2], $\gamma$, $\lambda$, and $\tau$ can be adjusted and used as hyperparameters, and each can be 0.

FIG. 3a is a schematic diagram showing a numeric vector including one or more elements that are biomarker candidates in one embodiment of the present application, and FIG. 3b is an explanatory diagram explaining the configuration and role of the corresponding numeric vector.

As shown in FIG. 3a, the numeric vector Z obtained through the encoder 20 may include one or more elements. Here, each of these elements becomes a biomarker candidate. As described below, each of these elements can be labeled and stored together with an attribute.

Here, at least some or all of the corresponding elements may be used to generate new biomarker candidate(s) by linearly or non-linearly combining them. In addition, at least some of the elements can be combined after transformation, such as polynomical, sigmoid, binary, log sqrt, etc., and additional transformations such as the above can be applied after the combination.

In one example, at least some of the elements of the numeric vector may be combined with existing known clinical biomarkers through linear or nonlinear transformation and used as biomarker candidates. In the process, a machine learning algorithm including a stepwise logistic regression using AIC may be used. The clinical biomarkers may include at least one of age, sex, SOFA score, Troponin I, and Pro-BNP level (value).

In one example, as described above, at least one of the mean, range, and standard deviation (SD) of one or more elements of a plurality of numeric vectors generated from the same input data may be included in the biomarker candidate.

As shown in FIG. 3b, the numeric vector Z may include a prediction vector, which is an output value associated with a specific task through supervised learning, and may also include an embedding vector (first embedding vector) obtained (extracted) in an intermediate step before obtaining the prediction vector.

In addition, the corresponding numeric vector Z may also include an embedding vector (second embedding vector) obtained through self-supervised learning or unsupervised learning.

The corresponding numeric vector Z may also include various basic patient data, such as the presence or absence of the patient's existing disease history or various measurement values.

Among the above bio vector candidates, the prediction vector obtained through patient data and supervised learning can be referred to as an explainable biomarker candidate. Such a prediction vector can include, for example, a probability value for the presence or absence of a specific state or a regression numeric value for a measurement value. Such an explainable biomarker candidate can be a clinical biomarker with clinical meaning. Such a clinical biomarker can be usefully used, for example, to select patients corresponding to the drug adaptation. In addition, it can be used by matching the drug effect pattern predicted by the drug structure with a known biomarker profile.

Meanwhile, the second embedding vector, which is an embedding vector obtained through self-supervised learning (or unsupervised learning), can be a morphological biomarker as a black box type biomarker candidate. Initially, the correlation with the clinical characteristics of patients cannot be known, but as research and analysis data on the correlation between them and clinical characteristics are accumulated, they become explainable, and when they become explainable to a certain level or higher, they can be included in the explainable biomarker candidate.

Meanwhile, in the case of the first embedding vector scattered in the middle of the supervised learning process, it can be classified as a black box type biomarker candidate whose clinical meaning is still difficult to explain because it corresponds to the calculation process before the final biomarker prediction. In this case, as research and analysis data on the correlation with clinical characteristics are accumulated, it becomes explainable, and if it becomes explainable to a certain level or higher, it can be included in the explainable biomarker candidate.

Meanwhile, in one example, the encoder 20 may use at least one of a Bayesian layer and a KL loss function to reduce correlation between multiple elements.

FIG. 3c is a schematic diagram illustrating a process for improving the embedding quality of each element of a numeric vector in one embodiment of the present application. To this end, a Bayesian layer may be added at the end of an encoder that creates an embedding vector, and the layer may be made to output a $\mu$ (mean) vector and a $\sigma$ (variance) vector that define a multivariate normal distribution used to extract embedding vector elements. Here, in order to make this multivariate normal distribution approximate a unit Gaussian (normal) distribution, the Kullback-Leibler (KL) divergence between the two multivariate distributions $N(\mu, \sigma)$ and $N(0, I)$ is calculated and reflected in the overall loss calculation Equation to minimize the KL divergence.

$$[\text{Equation 3}]$$
$$KL(\ N(\ \mu\ ,\ \sigma\ )\ |\ N(0,I)\ )$$

In the above [Equation 31, KL($\bullet|\bullet$) refers to the Kullback-Leibler divergence and N($\bullet$) refers to the normal distribution. If the encoder is trained in this way, the correlation between the elements of the embedding vector can be reduced and the unique characteristics of each element can be further highlighted.

Next, screening of biomarkers is explained .

The screening unit 30 can screen bio vectors from the bio vector candidates through regression, decision tree, clustering, dimension reduction, supervised learning, etc. that select variables in various ways.

In one example, the screening unit 30 may group one or more elements that are biomarker candidates and then select them as a biomarker.

In the case of biomarker candidates that have undergone the mentioned supervised learning prediction, they can be interpreted on their own and can be screened directly as biomarkers for each trained prediction task.

Meanwhile, in the case of numeric vector Z that is not related to supervised learning, grouping and selection can be performed by using one or more processes, such as a clustering process based on similarity or a selection process using a regression formula, alone or in combination.

FIG. 4 is a schematic diagram illustrating selection by clustering during a biomarker screening process in one embodiment of the present application. FIG. 4a is a diagram illustrating each individual cluster, and FIG. 4b is a diagram illustrating a numeric array (matrix) composed by extracting m candidate biomarkers from n subjects (or samples).

When applying a clustering technique to the array illustrated in Fig. 4b to assign m biomarker candidates to a smaller number of groups, each group corresponds to each cluster illustrated in 4a. In this case, candidate biomarkers belonging to one cluster can be presumed to be biomarkers with similar tendencies, and if there is a known explainable biomarker in this cluster (e.g., heart failure factor or hyperkalemia factor in Fig. 4a), it can be presumed that other biomarkers will also have similar tendencies based on this. The clustering algorithm used here can be any clustering method that can be commonly used in data mining.

Meanwhile, in one example, the screening unit 30 may store one or more elements of the numeric vector that is the biomarker candidate together with attribute information .

For example, even if there is an empirical interpretation through experiments for numeric vector elements that are morphological biomarker candidates, they can be used by tagging them as attributes of each element.

Meanwhile, in one embodiment, when the screening unit 30 wants to select some of the elements as biomarkers, the values to be predicted can be set in advance, each of the plurality of biodata can be labeled, and these can be selected as biomarkers through a regression equation.

The regression equation can use a logistic regression equation when predicting a binary event, and can use a regression equation when predicting a general number. This regression equation (R1) can be written as in the following [Equation 4],

$$[\text{Equation } 4]$$
$$Q1 = af(Z) + c$$

Here, f can be a linear or nonlinear transformation function including an identity function that does not do any processing, a is the effect size coefficient, and c is the intercept.

Using this regression equation, if the p value indicating statistical significance (or the value corrected by the Bonferroni correction method) is less than a preset value, and/or the absolute value of the effect size coefficient a is greater than a preset value, the corresponding biomarker candidate Z can be selected as a biomarker.

The numeric vector element values ($z_1$ , $z_2$, $z_3$,,) selected in this manner can be further screened as follows.

That is, the selected numeric vectors can be selected as biomarkers by using the LASSO regression method or the Elastic net regression method as numeric vectors whose coefficient values do not become 0.

Meanwhile, the regression method described above can be mainly used to predict specific values or events, but an effect modification method can be performed to predict the effect of exposure to a drug or the effect of exposure to a specific treatment, and in this method, exposure to a drug or treatment can be included in the regression equation.

The regression equation in this case may be , for example, the following [Equation 5] .

$$[\text{Equation } 5]$$
$$Q2 = a \cdot z_n \cdot \text{exposure} + b \cdot z_n + c \cdot \text{exposure} + d$$

The exposure may be a binary ( i.e. 0 or 1) or other numeric value relating to exposure to a drug effect or exposure to a specific treatment.

Through the above regression equation Q2, the biomarker candidate Z can be selected from the biomarker candidate vector Z by evaluating whether the effect modification of the exposure target is statistically significant. In other words, the effect size is the absolute value of a, and the effect size value or the p value (used for statistical significance evaluation) of the effect size value [or the value corrected using a correction method such as Bonferroni correction] can be used to check whether the interaction between the biomarker and the exposure is statistically significant and select it. That is, in the interaction term, an $a \cdot z_n \cdot$exposure, in the equation above, if the p value (or the value corrected in a manner such as Bonferroni correction) generally reported in regression analysis is less than a preset value, and/or the absolute value of the coefficient a, which is the effect size, is greater than or equal to a preset value, the corresponding biomarker candidate can be selected as an effect modification biomarker for a specific exposure.

The biomarker candidate $z_n$ value in [Equation 5] can be used after passing it through a linear or non-linear transformation function including an identity function, as shown in [Equation 5] . In other words, the regression equation Q3 of [Equation 6] can be used.

$$Q3 = \text{exposure } (a_1f_1(z_1) + a_2f_2(z_2) + ... + a_nf_n(z_n) + b_1g_1(z_1) + b_2g_2(z_2) + ... + b_ng_n(z_n) + c \cdot \text{exposure} + d \qquad \text{[Equation 6]}$$

Here, the f and g functions may be linear or nonlinear transformation functions including an identity function. The regression analysis method used here can be either the Lasso regression method or the Elastic net regression method as mentioned above, and also Akaike Information Criterion (AIC) and Bayesian Information Criterion (BIC)-based regression methods can be used.

Here, the weight decay of the Lasso regression method or the Elastic net regression method decay) related coefficients can be adjusted with hyperparameters.

Akaike Information Criterion (AIC) and Bayesian Information Criterion (BIC)-based regression methods are methods for selecting a subset of predictor variables in a multiple regression equation, where the set of variables with the lowest AIC or BIC value is used in the regression equation. This reduces the number of variables, simplifies the model, and maintains the regression model fit to the data.

Through the above process, a plurality of biomarker candidates $z_l$ to $z_n$ can be evaluated simultaneously. That is, in the case of the Lasso regression method or the Elastic Net regression method, a set of biomarker candidates Z' corresponding to the subset of terms a1 to an that are not 0 can be selected, and in the case of the AIC/BIC-based regression method, a vector Z" can be selected, which is a set of candidates selected based on the statistical validity of the interaction term included in the final regression equation (the previously mentioned p value, Bonferroni corrected p value).

In one example, the sum of the interaction terms included in the final regression equation can be used as a new biomarker candidate. That is, for example, in the case of the aforementioned [Equation 6], the value (numeric value) of

$$a'_1 \cdot f_1(z_1) \ + \ a'_2 \cdot f_2(z_2) \ + \ \cdots \ + \ a'_n \cdot f_n(z_n)$$

corresponding to the sum of the interaction terms of the final obtained regression equation can be used as a new biomarker.

FIG. 5 is a schematic diagram of a process for generating an effect modifier biomarker m that indicates the effect of a drug or treatment during a biomarker screening process using an artificial neural network in one embodiment of the present application, and a biomarker n that is unrelated to the effect modification. FIG. 5a illustrates a case where a numeric vector Z passes through one neural network, and FIG. 5b illustrates a case where a numeric vector Z passes through different neural networks.

As shown in FIGS. 5a and 5b, the numeric vector Z can be provided as m and n valuesby passing through one or more artificial neural networks 31, can go through a normalization process 32 in this process, and can pass through a network composed of transformation functions such as sigmoid, identity, and ReLU functions.

Fig. 5a shows that a neural network 31 through which a numeric vector Z passes has two heads at one bottom, where the two heads can have different normalization processes 32 and network structures 31. Fig. 5b shows that a neural network 31 through which a numeric vector Z passes can be trained as two different neural networks, each having a bottom and a head, with separate independent networks and hyperparameter sets.

In one example, various artificial neural networks (ANNs) including MLP can be applied as the artificial neural network 31.

The biomarkers m and n obtained in this way can be used as input values for the following regression equation [Equation 7].

$$[\text{Equation 7}]$$
$$Q4 = a \cdot m + b \cdot m \cdot \text{exposure} + c \cdot \text{exposure} + d \cdot n + e$$

The above Q4 is a predicted numeric value for the target (a treatment outcome, for example, whether to survive within a certain period, represented as 0 or 1, or a numeric value related to the amount or intensity of medication or treatment) of the exposure (a predicted numeric value, for example, whether to survive within a certain period, represented as 0 or 1, or a numeric value, such as the amount of weight loss due to treatment), and by applying the above Q4 to a generalized linear model, the artificial neural network can be trained to minimize the difference (i.e., loss) between the predicted numeric value and the target value, thereby creating an artificial neural network that generates an effect modifier biomarker m. This biomarker m allows for estimating a treatment effect (corresponding to the b·m·exposure term of Q4) that is affected by the patient condition, rather than a treatment effect (corresponding to the c·exposure term of Q4) that is unrelated to the patient condition that a specific exposure has.

Here, the loss function for reducing the difference (loss) between the predicted numeric value and the target value can be a cross entropy function or a focal loss function if the exposure is a binary variable, and a MSE function if the exposure is a numeric variable. However, various loss functions can be selected and used according to the problem to be solved.

In one example, the parameters of the regression equation and the neural network described above are optimized together during the training process, and various optimization algorithms used in neural network training can be applied for this purpose, and the selection is determined based on the final performance of the model..

Alternatively, an effect modifier can be created in the following way.

FIG. 6 is a schematic diagram illustrating a process for measuring effect modification during a biomarker screening process in one embodiment of the present application.

As shown in Fig. 6, when assuming a function h (Z, exposure) that takes a numeric vector Z and, for example, a binary exposure as input and predicts a patient treatment outcome, two functions can be assumed in the function h when the exposure is 0 (no exposure) or 1 (exposure). That is, g (Z) corresponding to h (Z, 0) when the exposure is 0, and f (Z) corresponding to h (Z, 1) when the exposure is 1 can be generated. Here, when the Z vector is displayed on the X-axis (the Z vector can be multidimensional, but is simplified to be processed as one-dimensional and displayed on the X-axis) and the outcome is displayed on the Y-axis, a function according to each exposure can be depicted. Here, the outcome can be, for example, the occurrence probability of a predicted event (e.g., death), the frequency (e.g., the frequency of seizures), or the numeric value (e.g., weight change), etc.

Meanwhile, a separate function m(Z) can be defined that defines the treatment effect, such as the difference between f(Z) in the case of exposure 1, i.e., exposure 0, i.e., g(Z) (or the ratio between exposure 1 and exposure 0, the odds ratio, the relative risk, etc., and the calculation method is not limited thereto), with the numeric vector Z as the X-axis and the effect size as the Y-axis. Such m(Z) can be calculated to show the aspect of the treatment effect changing according to the change in vector Z, as shown in Fig. 6 (bottom), so that it can nonlinearly show the effect modifier characteristic of vector Z without creating a separate biomarker. For example, if m(Z) is defined as f(Z)-g(Z), then it can be predicted that when the numeric vector Z is within a certain range, it will have a strong positive effect for the same treatment (e.g., recovery from a disease), while when it is within another certain range, it will have a strong negative effect (e.g., worsening of a disease).

In one example, the f, g, and h functions may be a machine learning algorithm, for example, but is not limited to various regression models, neural networks, tree ensembles, SVM, gradient boost, MLP , etc. Additionally, known methods for improving the performance of each function may be used.

FIG. 7 is a schematic diagram showing a process for finding attributes of a biomarker during a biomarker screening process in one embodiment of the present application. FIG. 7a is a schematic diagram summarizing the characteristics of a numeric vector Z, in which m biomarker candidates belonging to the numeric vector Z in an m x n matrix each have n attribute vectors X.

FIG. 7b is a diagram explaining a case where all biomarker candidates are divided into elements of a morphological numeric vector Z', a clinical numeric vector Z", and a phenotype numeric vector Z‴, and FIG. 7c is a diagram illustrating obtaining an attribute vector of a morphological numeric vector based on similarity.

Feature vector X may be n clinical characteristics (e.g., association with a specific disease, association with prognosis, drug effect or side effect) of each biomarker as shown in Fig. 7a, or may be measurements of biomarker candidates obtained from n patient cases as shown in Fig. 7b. Feature vectors X existing for each biomarker candidate element can be stored as attributes of each biomarker, and can be used to evaluate the similarity between attributes or cluster biomarkers.

If the biomarker feature vector X corresponds to measurements obtained from n patient cases, it can be used to generate similarity-based features (attributes) for each of the morphological biomarker candidates. As shown in Fig. 7c, this is done by repeatedly calculating the similarity (pairwise vector distance/similarity) between a vector X created by extracting a specific morphological biomarker belonging to an element of Z' from a series of patient cases and a vector X of explanatory (Z' [clinical] or Z" [phenotype] biomarkers) biomarkers extracted from the same cases, thereby extracting multiple similarity scores from one morphological biomarker candidate and obtaining a new similarity feature vector X'. This similarity feature vector X' can also be stored as an attribute of the corresponding biomarker, and can be used to evaluate the similarity between attributes or cluster biomarkers.

In this entire process, if the size (n) of the feature vector is too large, the m x n matrix may undergo a dimension reduction process. For example, the dimension may be reduced using at least one of the known dimension reduction techniques, such as PCA, LDA, SVD, and NMF.

In one example, a numeric vector Z can be selected as a biomarker through a clustering process. For example, various clustering techniques such as K means, K means+ + , TDA (topological data analysis), TsNE, and Mixture model can be used to convert a numeric vector Z into a K-class categorical variable (where K is the number of clusters), which can then be used as a biomarker. For example, by extracting numeric vectors Z in the same way from n patients to create training data, and using this to train algorithms that define similar clusters based on vector Z, we can then apply this algorithm to a new patient to find out which cluster this patient belongs to. If we know the characteristics (statistics) of each cluster, we can apply this to a new patient to estimate the characteristics of the new patient, and we can increase the prediction ability by converting the affiliation of a specific cluster into a K-hot vector and adding it as input data for a new prediction model.

FIG. 8 is a schematic diagram illustrating an artificial intelligence-based biomarker selection method in one embodiment of the present application. FIG. 8a is a schematic diagram illustrating each step of the method, and FIG. 8b is a schematic diagram showing a numeric vector, which are biomarker candidates obtained in the process of FIG. 8a, and selecting a biomarker therefrom to obtain a biomarker, particularly in the case of using electrocardiogram data.

As shown in FIG. 8a , the artificial intelligence-based biomarker selection method includes a step (S100) of acquiring data, an encoding step (S200) of encoding the biodata to generate a numeric vector including one or more elements that are candidate biomarkers, and a biomarker screening step (S300) of screening a biomarker from one or more elements of the numeric vector.

The final screened biomarkers obtained from this can be utilized in various ways, such as prognostic biomarkers, predictive biomarkers, monitoring biomarkers, and surrogate biomarkers.

Here, a prognostic biomarker is a biomarker that predicts the prognosis of a patient, a predictive biomarker is a biomarker that indicates whether a drug/treatment will be effective for the patient, a monitoring biomarker is a biomarker that observes whether a patient is experiencing problems such as side effects of a drug, and a surrogate marker biomarker is a biomarker that is measured as a substitute to indirectly see the risk of an event when it is difficult to directly observe the event that we need to observe.

FIG. 8b is a schematic diagram explaining each step in the case of inputting electrocardiogram data. As described above, numeric vectors, which are biomarker candidates, are obtained through encoding (S200), and biomarkers are selected from these through regression methods (S300) and used as final biomarkers.

The exemplary technologies of the present application described above can be applied to all types of diseases other than cardiovascular diseases. In addition, the numeric vectors described above can be used in general artificial intelligence-based medical platforms for diagnosing and predicting various diseases, and can be used as input values that can be utilized in various artificial intelligence algorithms.

Description of Experimental Example

FIGS 9a to 9c are tables and graphs showing experimental data for developing a new electrocardiogram (ECG) biomarker for predicting the risk of death in COVID-19 infection patients using a numeric vector Z extracted from an ECG analysis encoder using 12-lead ECG data, and evaluating its sensitivity, specificity, etc., in an experimental example of the present invention.

Referring to FIG. 9a, in one experimental example, a total of 167 COVID-19 patients admitted to Seoul National University Bundang Hospital were studied. FIG. 9a shows the patient's basic data, and the death outcome ("Poor") was defined as in-hospital death or application of ECMO.

From the first ECG of the study subjects upon admission, a CNN-based ECG encoder trained by integrating self-supervised learning and supervised learning techniques was used to extract biomarker candidates consisting of a total of 244 elements.

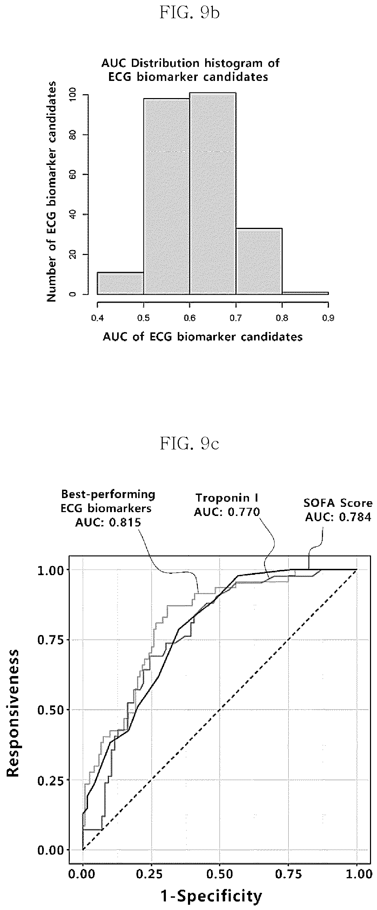

FIG. 9b shows the individual prediction performance of the 244 extracted biomarker candidates as a histogram.

As shown in FIG. 9b , the individual prediction performances of the 244 biomarker candidates extracted varied based on AUC, and the highest prediction performance was 0.815. In addition, the sensitivity was 87.2%, the specificity was 69.2%, the positive predictive value was 52.6%, and the negative predictive value was 93.3%. Referring to FIG. 9c, the AUC of the best-performing biomarker candidate extracted from the present invention was statistically significantly higher (P<0.05) than the SOFA score (0.784) or Troponin I blood concentration (0.770), which are clinical biomarkers known to be closely related to patient prognosis in clinical settings. During screening, the screening unit can select the corresponding biomarker candidate as a biomarker.

Additionally, the five clinical data (age, sex, SOFA score, Troponin I blood concentration, and Pro-BNP) including the ECG biomarker candidate and the clinical biomarker SOFA score and Troponin I blood concentration were nonlinearly rearranged through a machine learning algorithm (Stepwise logistic regression using AIC) to create a new biomarker candidate and evaluate its performance. To prevent overfitting of the model, 4-fold cross-validation was applied to obtain the final prognostic biomarker. The AUC of the new biomarker obtained in this way was 0.820, which was statistically significantly higher than the performance of the AUC of 0.694 when the five clinical data were combined without using the ECG biomarker (p<0.05).

The new prognostic biomarker created in this way also showed higher predictive performance than the SOFA score or Troponin I blood concentration, which are known to be closely related to patient prognosis in clinical practice.

In conclusion, the candidates of electrocardiogram biomarkers extracted using the electrocardiogram encoder trained by integrating the self-supervised learning and supervised learning techniques of the present invention through the above experimental examples showed various degrees of predictive ability, and it was found that by combining these with general clinical biomarkers, a new biomarker with high performance can be created simply by applying a regression model.

The method according to the embodiments described above and the operation of the device for performing the method may be at least partially implemented as a computer program and recorded on a computer-readable recording medium.

For example, it can be implemented together with a program product consisting of a computer-readable medium including program code, which can be executed by a processor to perform any or all of the steps, operations, or processes described.

The computer may be any device, such as a desktop computer, a laptop computer, a notebook, a smart phone, or the like, or may be integrated. The computer is a device having one or more alternative and special purpose processors, memory, storage, and networking components (either wireless or wired). The computer may run an operating system, such as, for example, a Windows compatible operating system from Microsoft, Apple OS X or iOS, a Linux distribution, or Google's Android OS.

The computer-readable recording medium includes all types of recording devices that store data that can be read by a computer. Examples of computer-readable recording media include ROM, RAM, CD-ROM, magnetic tape, floppy disk , and optical data storage devices. Additionally, computer-readable recording media may be distributed across computer systems connected to a network, and computer-readable codes may be stored and executed in a distributed manner. Additionally, functional programs, codes, and code segments for implementing this embodiment can be easily understood by those skilled in the art to which this embodiment belongs.

The present invention examined above has been described with reference to the embodiments shown in the drawings, but these are merely illustrative examples, and a person skilled in the art will understand that various modifications and modifications of the embodiments are possible therefrom. However, such modifications should be considered within the technical protection scope of the present invention. Therefore, the true technical protection scope of the present invention should be determined by the technical spirit of the attached patent claims.

**Industrial Applicability**

The present application relates to an artificial intelligence-based biomarker selection device and method that can develop companion diagnostic biomarkers that can be used for new drug development, precision medicine, disease diagnosis, and treatment optimization for various diseases, including diseases for which existing biomarkers are absent, such as cardiovascular diseases.

This application can be used for the discovery of various biomarkers that can be used for drug discovery, such as cardiovascular drug development, or prognosis prediction. Accordingly, it can be effectively utilized as a pharmaceutical platform for new drug development or a research platform for precision medicine, disease diagnosis, and treatment optimization.

**Claims**

1. An artificial intelligence-based biomarker selection device, comprising:

   an acquisition unit for acquiring biodata;
   an encoder for receiving the biodata and calculating a numeric vector including one or more elements which are biomarker candidates; and
   a biomarker screening unit for screening biomarkers from one or more elements of the numeric vector.

2. The artificial intelligence-based biomarker selection device of claim 1, wherein the biodata is two or more biodata with different or identical modalities.

3. The artificial intelligence-based biomarker selection device of claim 2, wherein the biodata comprises at least one of signal data and image data.

4. The artificial intelligence-based biomarker selection device of claim 3, wherein the encoder is trained in a way to project each numeric vector produced by position embedding a plurality of signals and/or images onto a common space using a separate artificial neural network trained together, and find a positive pair that is a pair within a given time range of the same subject; and/or a negative pair that is a pair outside the given time range of the same subject or pairs between different subjects; based on the similarity between the numeric vectors.

5. The artificial intelligence-based biomarker selection device of claim 4 , wherein the positive pair or negative pair is a pair including one or more of a pair between signals; a pair between images; and a pair between signals and images.

6. The artificial intelligence-based biomarker selection device of claim 4, wherein the encoder comprises NCE loss as a loss function.

7. The artificial intelligence-based biomarker selection device of claim 6, wherein the loss function further comprises at least one of MIL NCE loss and supervised task loss.

8. The artificial intelligence-based biomarker selection device of claim 7, wherein the supervised task is a task related to prediction, which is a task of predicting one or more of binary, multi-category, and numeric values simultaneously or sequentially.

9. The artificial intelligence-based biomarker selection device of claim 4, wherein the encoder is trained using a plurality of signals and/or images, and the plurality of signals and/or images are subjected to data augmentation processing by applying original signal and/or imaging transformation.

10. The artificial intelligence-based biomarker selection device of claim 9, wherein the encoder comprises a first encoder for receiving a first biodata set subjected to data augmentation processing; and a second encoder for receiving a second biodata set subjected to data augmentation processing; and the first biodata set and the second biodata set are of the same modality, and the second encoder is a momentum encoder that shares the same weight as the first encoder or averages the temporal changes of the first encoder.

11. The artificial intelligence-based biomarker selection device of claim 10, wherein the first encoder may or may not include an MLP layer, and the second encoder may or may not perform clustering, which replaces the output numeric vector with an embedding numeric vector representative value.

12. The artificial intelligence-based biomarker selection device of claim 11, wherein at least one of the first encoder and the second encoder calculates a similarity loss between positive pairs by a similarity function, and/or at least one of the first encoder and the second encoder calculates dissimilarity loss between negative pairs by a dissimilarity function.

13. The artificial intelligence-based biomarker selection device of claim 1, wherein the encoder comprises at least one of a clinical encoder and a morphologic encoder, the clinical encoder is trained by supervised learning, and the morphologic encoder is trained by unsupervised learning.

14. The artificial intelligence-based biomarker selection device of claim 13, wherein the encoder uses a numeric vector that is a concatenation of a numeric vector produced from a clinical encoder and a numeric vector produced from a morphological encoder, and is trained by multi-task learning that integrates supervised learning and unsupervised learning.

15. The artificial intelligence-based biomarker selection device of claim 1, wherein at least one of the mean, range and standard deviation (SD) of some or all of one or more elements of each numeric vector repeatedly generated from the encoder is included in a biomarker candidate.

16. The artificial intelligence-based biomarker selection device of claim 1, wherein the biomarker screening unit stores one or more elements of the numeric vector, which is the biomarker candidate, together with attribute information.

17. The artificial intelligence-based biomarker selection device of claim 1, wherein at least some of the elements of the numeric vector are combined through linear or nonlinear transformation and used as biomarker candidates.

18. The artificial intelligence-based biomarker selection device of claim 17, wherein at least some of the elements of the numeric vector are combined through linear or nonlinear transformation with a clinical biomarker and used as a biomarker candidate.

19. The artificial intelligence-based biomarker selection device of claim 1, wherein values obtained through the supervised task related to the prediction of the encoder are concatenated into the numeric vector.

**20.** The artificial intelligence-based biomarker selection device of claim 1, wherein the numeric vector includes a morphological numeric vector, a clinical numeric vector, and basic patient data.

**21.** The artificial intelligence-based biomarker selection device of claim 1, wherein the biomarker screening unit screens regression, decision tree, clustering, dimension reduction, and supervised bio vectors.

**22.** The artificial intelligence-based biomarker selection device of claim 20, wherein the biomarker screening unit groups one or more elements that are biomarker candidates and selects them as biomarkers.

**23.** The artificial intelligence-based biomarker selection device of claim 22, wherein the biomarker screening unit selects one or more elements of a numeric vector related to a supervised task related to prediction as biomarkers, and/or selects one or more elements of a numeric vector unrelated to a supervised task related to prediction as biomarkers using a clustering technique based on similarity with predictor elements related to a supervised task related to prediction.

**24.** The artificial intelligence-based biomarker selection device of claim 22, wherein the biomarker screening unit selects a numeric vector, which is a biomarker candidate, as a biomarker using the following regression equation, [Equation 4],

$$[\text{Equation 4}]$$
$$Q1 = af(Z) + c$$

wherein f is a linear or nonlinear transformation function including an identity function that has not been specifically processed, absolute value of the coefficient a represents the effect size, c is an intercept, whether the numeric vector Z is statistically significant for the result Q1 is evaluated in the regression equation of the [Equation 4], and if the absolute value of the coefficient a, which is the effect size, is greater than or equal to a preset value and the p-value of a is less than or equal to a preset value, the numeric vector Z is selected as a biomarker.

**25.** The artificial intelligence-based biomarker selection device of claim 24, wherein the biomarker screening unit further selects numeric vectors whose coefficient values do not become 0 as biomarkers through the Lasso regression method and/or the Elastic net regression method for a plurality of selected numeric vectors.

**26.** The artificial intelligence-based biomarker selection device of claim 22, wherein the biomarker screening unit selects biomarkers using the following regression equation [Equation 5],

$$[\text{Equation 5}]$$
$$Q2 = a \cdot z_n \cdot \text{exposure} + b \cdot z_n + c \cdot \text{exposure} + d$$

wherein a, b, and c are coefficients, d is an intercept, exposure is a binary or other numeric variable value regarding exposure to a specific drug or treatment,
biomarker candidates $z_1 .. z_n$ are evaluated to determine whether the effect modification of the target outcome of the exposure is statistically significant in the regression equation of the above [Equation 5], and if the absolute value of the coefficient a, which is the effect size, is greater than or equal to a preset value and the p-value of a is less than or equal to a preset value, the biomarker $z_n$ is selected as a biomarker.

**27.** The artificial intelligence-based biomarker selection device of claim 26, wherein the biomarker screening unit selects biomarkers using the following regression equation [Equation 6] for one or more biomarker candidates $Z_1 , Z_2 .. Z_n$,

$Q3 = \text{exposure} (a_1 f_1(z_1) + a_2 f_2(z_2) + ... + a_n f_n(z_n) + b_1 g_1(z_1) + b_2 g_2 (z_2) + ... + b_n g_n(z_n) + c \cdot \text{exposure} + d$  [Equation 6]

Wherein f function and g function are linear or nonlinear transformation functions including identity function, $a_1 , a_2 .. a_n, b_1 , b_2 .. b_n$, and c are coefficients, d is an intercept, exposure is a binary or other numeric variable value regarding exposure to a specific drug or treatment, a new Z' vector consisting of a set of biomarker candidates with non-zero coefficients from $a_1$ to $a_n$ is selected as a biomarker when using Lasso regression method or Elastic net regression method as a regression method,
and a new Z" vector consisting of a set of biomarker candidates selected based on the statistical validity of the interaction term included in the final regression equation is selected as a biomarker when using AIC and BIC-based regression method as a regression method.

28. The artificial intelligence-based biomarker selection device of claim 27, wherein the $a'_1 \cdot f_1(z_1) + a'_2 \cdot f_2(z_2) + \cdots + a'_n \cdot f_n(z_n)$ value (numeric value), which is the sum of the interaction terms in the regression equation of the [Equation 6], is additionally used as a new biomarker.

29. The artificial intelligence-based biomarker selection device of claim 22, wherein the biomarker screening unit generates an effect modifier using the following regression equation [Equation 7],

$$[\text{Equation 7}]$$
$$Q4 = a \cdot m + b \cdot m \cdot \text{exposure} + c \cdot \text{exposure} + d \cdot n + e$$

wherein a, b, c, d are coefficients, e is an intercept, exposure is a binary or other numeric variable value regarding exposure to a specific drug or treatment, the above m is an effect modifier biomarker that informs the effect of a specific drug or treatment selected by passing the numeric vector Z through an artificial neural network, n is a biomarker unrelated to effect modification, and
the predicted numeric value Q4 obtained from the regression equation of the [Equation 7] is applied to a generalized linear model, and the artificial neural network is trained to minimize the difference between the predicted numeric value and the target value, thereby generating an m value, which is an effect modifier biomarker, and selecting it as a biomarker.

30. The artificial intelligence-based biomarker selection device of claim 29, wherein the difference between the predicted numeric value and the target value is calculated by using the cross entropy loss function or the focal loss function when the exposure is a binary variable, and by using the MSE function when the exposure is a numeric variable..

31. The artificial intelligence-based biomarker selection device of claim 22, wherein the biomarker screening unit receives a numeric vector Z and a binary exposure as inputs and selects a biomarker from at least one of the difference between two function values when the exposure is 0 or 1, the ratio of the two function values, the odds ratio, and the relative risk in a function h (Z, exposure) that predicts a patient treatment outcome.

32. The artificial intelligence-based biomarker selection device of claim 16, wherein the biomarker screening unit configures an m x n matrix composed of m biomarker candidates and n feature vectors X, and the feature vector X may be a feature of each of the m biomarker candidates, and one or more elements of the feature vector X existing for each of the m biomarker candidates in the m x n matrix are stored as attributes of the biomarker to evaluate the similarity between attributes or to cluster the biomarkers.

33. The artificial intelligence-based biomarker selection device of claim 32, wherein the matrix is used by dimension reduction.

34. The artificial intelligence-based biomarker selection device of claim 32, wherein the feature vector X existing for each of the m biomarker candidates includes a morphological numeric vector, a clinical numeric vector, and a phenotype vector, and a vector composed of elements such as the similarity between the morphological numeric vector and the clinical numeric vector and/or the similarity between the morphological numeric vector and the phenotype vector is used as an attribute of the morphological numeric vector.

35. The artificial intelligence-based biomarker selection device of claim 1, wherein the encoder uses at least one of a Bayesian layer and a KL loss function to reduce the correlation between a plurality of elements.

36. The artificial intelligence-based biomarker selection device of claim 1, wherein the biomarker is a predictive biomarker that can distinguish between responders and non-responders to a specific drug, and/or a prognostic biomarker that can predict the prognosis of a disease.

37. The artificial intelligence-based biomarker selection device of any one of claims 1 to 36, wherein the biodata is electrocardiogram (ECG) data.

38. The artificial intelligence-based biomarker selection device of claim 37, wherein the biomarker is a biomarker related to cardiovascular disease.

39. An artificial intelligence-based biomarker selection method, comprising: an encoding step for encoding biodata to calculate a numeric vector including one or more elements that are biomarker candidates; and a biomarker screening step for screening biomarkers from one or more elements of the numeric vector.

40. An artificial intelligence-based biomarker selection method of claim 39, wherein the encoding step is to train in a way to project each numeric vector produced by position embedding a plurality of signals and/or images onto a common space using a separate artificial neural network trained together, and find a positive pair that is a pair within a given time range of the same subject; and/or a negative pair that is a pair outside the given time range of the same subject or pairs between different subjects; based on the similarity between the numeric vectors, includes NCE loss as a loss function when training, and further includes at least one of MIL NCE loss and supervised task loss.

41. An artificial intelligence-based biomarker selection method of claim 39, wherein the encoding step is trained using different encoders using a plurality of signals or images of same modality,

> the different encoders share same weight or use a momentum encoder that averages the temporal changes of one encoder as another encoder, the encoding step may or may not include an MLP layer,
> the encoding step performs clustering that replaces the produced numeric vector with a representative value of an embedding numeric vector, or does not perform it, and
> the encoding step calculates a similarity loss between positive pairs by a similarity function, and/or calculates a dissimilarity loss between negative pairs by a dissimilarity function.

42. An artificial intelligence-based biomarker selection method of claim 39, wherein the biomarker screening step screens bio vectors from biomarker candidates through one or more of regression, decision tree, clustering, dimension reduction, and supervised learning.

43. An artificial intelligence-based biomarker selection method of claim 42, wherein the biomarker screening step selects one or more elements of a numeric vector related to a supervised task related to prediction as biomarkers, and/or selects one or more elements of a numeric vector unrelated to a supervised task related to prediction as biomarkers using a clustering technique based on similarity with predictor elements related to a supervised task related to prediction.

44. An artificial intelligence-based biomarker selection method of claim 42, wherein the biomarker screening step s to select a numeric vector as a biomarker candidate using the following regression equation [Equation 4],

$$[\text{Equation 4}]$$
$$Q1 = af(Z) + c$$

> wherein f is a linear or nonlinear transformation function including an identity function that has not been specifically processed, the absolute value of the coefficient a represents the effect size, c is an intercept,
> whether the numeric vector Z is statistically significant for the result Q1 is evaluated in the regression equation of the [Equation 4],
> and if the absolute value of the effect size coefficient a is greater than or equal to a preset value and the p-value of a is less than or equal to a preset value, the numeric vector Z is selected as a biomarker.

45. An artificial intelligence-based biomarker selection method of claim 42, wherein the biomarker screening step selects a biomarker using the following regression equation [Equation 5],

$$[\text{Equation 5}]$$
$$Q2 = a \cdot z \cdot exposure + b \cdot z_n + c \cdot exposure + d$$

> wherein a, b, and c are coefficients, d is an intercept, exposure is a binary or other numeric variable value regarding exposure to a specific drug or treatment,

the numeric vector Z, which is a biomarker candidate, evaluates whether the effect modification for the target result of the exposure is statistically significant in the regression equation of the [Equation 5], and if the absolute value of the coefficient a, which is the effect size, is greater than or equal to a preset value and the p-value of a is less than or equal to a preset value, the biomarker $z_n$ is selected as a biomarker.

46. An artificial intelligence-based biomarker selection method of claim 42, wherein the biomarker screening step is to select a biomarker using the following regression equation [Equation 6] for one or more biomarker candidates $z_1 , z_2 , .. z_n$,

$$Q3 = exposure.(a_1f_1(z_1) + a_2f_2(z_2) + ... + a_nf_n(z_n) + b_1g_1(z_1) + b_2g_2(z_2) + ... + b_ng_n(z_n) + c \cdot exposure + d \qquad \text{[Equation 6]}$$

wherein f function and the g function are linear or nonlinear transformation functions including an identity function, $a_1, a_2 .. a_n , b_1, b_2 .. b_n , c$ are coefficients, d is the intercept, exposure is a binary or other numeric variable value related to exposure to a specific drug or treatment,
a new Z' vector consisting of a set of biomarker candidates with non-zero coefficients from a1 to an is selected as a biomarker when using the Lasso regression method or the Elastic net regression method as a regression method,
a new Z" vector consisting of a set of biomarker candidates selected based on the statistical validity of the interaction terms included in the final regression equation is selected as a biomarker when using the AIC and BIC-based regression methods as the regression method,
and the sum of the interaction terms in the regression equation of the [Equation 6], $a'_1 \cdot f_1(z_1) + a'_2 \cdot f_2(z_2) + \cdots + a'_n \cdot f_n(z_n)$ (numeric value), is additionally used as a new biomarker.

47. An artificial intelligence-based biomarker selection method of claim 42, wherein the biomarker screening step generates an effect modifier biomarker using the following regression equation [Equation 7],

$$[\text{Equation 7}]$$
$$Q4 = a \cdot m + b \cdot m \cdot \text{exposure} + c \cdot \text{exposure} + d \cdot n + e$$

wherein a, b, c, and d are coefficients, e is an intercept, exposure is a binary or other numeric variable value regarding exposure to a specific drug or treatment,
the m is an effect modifier biomarker that informs the effect of a specific drug or treatment selected by passing the numeric vector Z through an artificial neural network, n is a biomarker unrelated to effect modification, the predicted numeric value Q4 obtained from the regression equation of the [Equation 7] is applied to a generalized linear model, and the artificial neural network is trained to minimize the difference between the predicted numeric value and the target value, thereby generating an effect modifier m value and selecting it as a biomarker,
and the difference between the predicted numeric value and the target value is the cross entropy loss function or the focal loss function when the exposure is a binary variable, and the MSE function when the exposure is a numeric variable.

48. An artificial intelligence-based biomarker selection method of claim 42, wherein the biomarker screening step receives a numeric vector Z and a binary exposure as inputs and selects a biomarker from at least one of the difference, ratio, odds ratio and relative risk between the two functions when the exposure is 0 or 1 in a function h (Z, exposure) that predicts a patient treatment outcome.

49. An artificial intelligence-based biomarker selection method of claim 39, wherein the biomarker screening step comprises an m x n matrix composed of m biomarker candidates and n feature vectors X,

the feature vector X may be a feature of each of the m biomarker candidates, and one or more elements of the feature vector X, which has n elements for each of the m biomarker candidates in the m x n matrix, are stored as attributes of the biomarkers to evaluate the similarity between attributes or cluster the biomarkers,
and the matrix is used by performing a dimension reduction.

50. An artificial intelligence-based biomarker selection method of claim 49, wherein the n feature vectors X existing for each of the m biomarker candidates include a morphological numeric vector, a clinical numeric vector, and a phenotype vector, and a vector composed of elements such as the similarity between the morphological numeric vector and the clinical numeric vector and/or the similarity between the morphological numeric vector and the phenotype vector is used as an attribute of the morphological numeric vector.

51. An artificial intelligence-based biomarker selection method of claim 39, wherein the encoding step uses at least one of a Bayesian layer and a KL loss function to reduce the correlation between a plurality of elements.

52. A computer program combined with hardware and stored in a medium to execute an artificial intelligence-based biomarker selection method according to any one of claims 39 to 51.

FIG. 1

Biodata

Biodata —10

Encoder —20

Biomarker
screening unit —30

prognostic biomarker
predictive biomarker
monitoring biomarker
surrogate biomarker

●
●
●
●

FIG. 2a

FIG. 2b

25

Patient 1

matching pair

non-matching pair

Patient 2

matching pair

● Signal data

▨ Image data

FIG. 3a

element

New numeric vectors
can be created by linear
or non-linear combination.

Numeric vector Z

FIG. 3b

Numeric vector Z

FIG. 3c

FIG. 4a

First cluster

Heart failure element

Second cluster

High potassium blood element

FIG. 4b

m Biomarker candidates

n Samples

Clustering

FIG. 5a

Numeric vector Z

Artificial neural network

31

32

32

33

33

m

n

FIG. 5b

Numeric vector Z

31

32

32

33

33

m

n

FIG. 6

FIG. 7a

n Samples

Feature
vector X

m Biomarker
candidates

Numeric vector Z

FIG. 7b

n Samples

Z ' morphologic
numeric vector

Z " Clinical
numeric vector

Z ''' Phenotype
numeric vector

FIG. 7c

Morphological
numeric vector
Zk

Similarity 1  Similarity 2  Similarity 3

• • • •

Similarity to clinical
numeric vectors

Similarity with
phenotype numeric vector

FIG. 8a

```
┌─────────────────────────┐
│   Biodata acquisition   │──── S100
└─────────────────────────┘
            │
            ▼
┌─────────────────────────┐
│    Biodata encoding     │──── S200
└─────────────────────────┘
            │
            ▼
┌─────────────────────────┐
│   Biomarker screening   │──── S300
└─────────────────────────┘
            │
            ▼
```

prognostic biomarker
predictive biomarker
monitoring biomarker
surrogate biomarker

FIG. 8b

prognostic biomarker
predictive biomarker
monitoring biomarker
surrogate biomarker

regression
decision tree
clustering
dimensional reduction
supervised trainin

S300

Biomarker candidates

Basic patient data
predictive vector
First embedding vector
Second embedding vector

Numeric vector Z

S200

Supervised learning
Unsupervised learning
Encoding

Electrocardiogram data

S100

| | Total (n=167) | Normal (n=120) | Death or ECMO (n=47) | p-value |
|---|---|---|---|---|
| Troponin I (Troponin I) (ng/ml) | 0.022 [0.008 - 0.100] | 0.013 [0.006 - 0.032] | 0.091 [0.022 - 0.257] | <0.001 |
| Pro-BNP (pg/ml) | 592.1 [147.1 - 2244.7] | 419.4 [107.3 - 1108.4] | 1909.1 [458.8 - 9415.5] | <0.001 |
| SOFA Score(SOFA score) | 3 [1 - 6] | 2 [1 - 5] | 6 [4 - 8] | <0.001 |
| Remdesivir | 102 (61.1%) | 67 (55.8%) | 35 (74.5%) | 0.041 |
| Dexamethasone | 114 (68.3%) | 76 (63.3%) | 38 (80.9%) | 0.045 |
| High flow nasal cannula | 82 (49.1%) | 50 (41.7%) | 32 (68.1%) | 0.004 |
| Mechanical ventilator | 70 (41.9%) | 30 (25.0%) | 40 (85.1%) | <0.001 |

FIG. 9a

FIG. 9b

AUC Distribution histogram of
ECG biomarker candidates

AUC of ECG biomarker candidates

FIG. 9c

## INTERNATIONAL SEARCH REPORT

| International application No. |
|---|
| **PCT/KR2023/001315** |

| A. | CLASSIFICATION OF SUBJECT MATTER |
|---|---|

**G16H 50/20**(2018.01)i; **G16B 40/20**(2019.01)i; **G16B 15/30**(2019.01)i; **A61B 5/00**(2006.01)i; **A61B 5/318**(2021.01)i; **G16H 10/20**(2018.01)i; **G16H 20/10**(2018.01)i; **G16H 50/50**(2018.01)i; **G01N 33/68**(2006.01)i

According to International Patent Classification (IPC) or to both national classification and IPC

| B. | FIELDS SEARCHED |
|---|---|

Minimum documentation searched (classification system followed by classification symbols)

G16H 50/20(2018.01); A61B 5/00(2006.01); A61B 5/24(2021.01); G06K 9/00(2006.01); G06K 9/62(2006.01); G06N 20/00(2019.01); G16B 20/00(2019.01); G16B 5/00(2019.01); G16H 50/30(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 바이오 마커(biomarker), 선별(screening), 바이오 데이터(bio data), 인코더 (encoder), 수치 벡터(numerical vector), 인공지능(artificial intelligence)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | WO 2020-260901 A1 (BIOS HEALTH LTD.) 30 December 2020 (2020-12-30)<br>See claims 1-62; and paragraphs [0050], [00113] and [00122]. | 1-3,13-18,20-22,35-39,42,51-52 |
| A | | 4-12,19,23-34,40-41,43-50 |
| Y | KR 10-2020-0068161 A (KONYANG UNIVERSITY INDUSTRIAL COOPERATION GROUP) 15 June 2020 (2020-06-15)<br>See abstract; claims 1-5; and paragraphs [0097] and [0130]. | 1-3,13-18,20-22,35-39,42,51-52 |
| A | KR 10-2021-0139195 A (LUNIT INC.) 22 November 2021 (2021-11-22)<br>See entire document. | 1-52 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **02 May 2023** | **03 May 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/001315** |

### C. DOCUMENTS CONSIDERED TO BE RELEVANT

| Category* | Citation of document, with indication, where appropriate, of the relevant passages | Relevant to claim No. |
| --- | --- | --- |
| A | US 2021-0350176 A1 (HOFFMANN-LA ROCHE INC.) 11 November 2021 (2021-11-11)<br>See entire document. | 1-52 |
| A | KR 10-2309002 B1 (INCHEON NATIONAL UNIVERSITY RESEARCH & BUSINESS FOUNDATION) 05 October 2021 (2021-10-05)<br>See entire document. | 1-52 |

Form PCT/ISA/210 (second sheet) (July 2022)

<table>
<tr><td colspan="7"><b>INTERNATIONAL SEARCH REPORT</b><br>Information on patent family members</td><td colspan="2">International application No.<br><br><b>PCT/KR2023/001315</b></td></tr>
</table>

| Patent document cited in search report | | | Publication date (day/month/year) | Patent family member(s) | | | Publication date (day/month/year) |
|---|---|---|---|---|---|---|---|
| WO | 2020-260901 | A1 | 30 December 2020 | AU | 2020-301632 | A1 | 10 February 2022 |
|  |  |  |  | CA | 3144315 | A1 | 30 December 2020 |
|  |  |  |  | CN | 114616632 | A | 10 June 2022 |
|  |  |  |  | EP | 3991181 | A1 | 04 May 2022 |
|  |  |  |  | JP | 2022-539080 | A | 07 September 2022 |
|  |  |  |  | KR | 10-2022-0037455 | A | 24 March 2022 |
|  |  |  |  | US | 2022-0254492 | A1 | 11 August 2022 |
| KR | 10-2020-0068161 | A | 15 June 2020 | KR | 10-2226875 | B1 | 11 March 2021 |
| KR | 10-2021-0139195 | A | 22 November 2021 | EP | 4152343 | A1 | 22 March 2023 |
|  |  |  |  | US | 2022-0037024 | A1 | 03 February 2022 |
|  |  |  |  | WO | 2021-230687 | A1 | 18 November 2021 |
| US | 2021-0350176 | A1 | 11 November 2021 | CN | 113454733 | A | 28 September 2021 |
|  |  |  |  | EP | 3938948 | A1 | 19 January 2022 |
|  |  |  |  | JP | 2022-527145 | A | 31 May 2022 |
|  |  |  |  | WO | 2020-182710 | A1 | 17 September 2020 |
| KR | 10-2309002 | B1 | 05 October 2021 | KR | 10-2021-0052855 | A | 11 May 2021 |

Form PCT/ISA/210 (patent family annex) (July 2022)

**REFERENCES CITED IN THE DESCRIPTION**

**Patent documents cited in the description**

• KR 1020220013491